(19) Europäisches Patentamt
European Patent Office
Office européen des brevets

(11) **EP 2 473 101 B1**

(12) **EUROPÄISCHE PATENTSCHRIFT**

(45) Veröffentlichungstag und Bekanntmachung des Hinweises auf die Patenterteilung:
**15.04.2020 Patentblatt 2020/16**

(21) Anmeldenummer: **10760273.2**

(22) Anmeldetag: **02.09.2010**

(51) Int Cl.:
**A61B 5/021** *(2006.01)* **A61B 5/053** *(2006.01)*

(86) Internationale Anmeldenummer:
**PCT/EP2010/062873**

(87) Internationale Veröffentlichungsnummer:
**WO 2011/026899 (10.03.2011 Gazette 2011/10)**

(54) **VORRICHTUNG ZUR NICHT-INVASIVEN BESTIMMUNG DES ARTERIELLEN BLUTDRUCKS**

DEVICE FOR THE NON-INVASIVE DETERMINATION OF ARTERIAL BLOOD PRESSURE

DISPOSITIF POUR LA DÉTERMINATION NON INVASIVE DE LA TENSION ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **03.09.2009 AT 13912009**

(43) Veröffentlichungstag der Anmeldung:
**11.07.2012 Patentblatt 2012/28**

(73) Patentinhaber: **Heller, Arnulf**
**8043 Graz (AT)**

(72) Erfinder: **Heller, Arnulf**
**8043 Graz (AT)**

(74) Vertreter: **Margotti, Herwig Franz**
**Schwarz & Partner**
**Patentanwälte**
**Wipplingerstrasse 30**
**1010 Wien (AT)**

(56) Entgegenhaltungen:
**US-A- 4 646 754 US-A- 5 309 916**
**US-A1- 2008 009 759**

## Beschreibung

Die Erfindung betrifft eine Vorrichtung zur nicht-invasiven Bestimmung des arteriellen Blutdrucks eines menschlichen oder tierischen Körpers, umfassend zumindest eine Bioimpedanz-Messeinrichtung mit mehreren Elektrodenpaaren zur Erfassung der durch einen eingeprägten Wechselstrom verursachten Admittanzmesssignale an zumindest einem ersten Abschnitt des Körpers, wobei die erfassten Admittanzmesssignale einem Summensignal aus Signalanteilen einer Puls-Admittanz, einer Atmungs-Admittanz sowie einer Basis-Admittanz entsprechen, sowie zumindest eine Einrichtung zur nicht-invasiven Blutdruckmessung.

Die Impedanzkardiographie wird seit längerem erfolgreich zur nicht-invasiven, also unblutigen Messung bestimmter hämodynamischer Parameter des Herzens wie des Schlagvolumens, oder des Herz-Zeit-Volumens eingesetzt. Diese Parameter spielen bei der Überwachung von Intensivpatienten eine entscheidende Rolle.

Im Gegensatz zu invasiven Methoden, in welchen ein Katheter gelegt werden muss, nutzt man bei der Impedanzkardiographie Schwankungen des Widerstandes über dem Thorax während einer Herzperiode. Dazu wird ein kleiner, konstanter Messstrom in den Körper geleitet und über die Spannungsänderung die Impedanzänderung bestimmt. Aus dieser Kurve lassen sich dann die zuvor genannten hämodynamischen Parameter bestimmen. Im Allgemeinen bietet die Impedanzkardiographie eine gute Korrelation zu den invasiven Standardmethoden, ist aber aufgrund der Nicht-Invasivität weitaus komplikationsärmer. Ein weiterer Vorteil der Impedanzkardiographie entsteht durch die Beat-to-beat-Messung des Schlagvolumens. Damit kann man in Echtzeit die Entwicklung des Patienten beurteilen und überwachen.

Bei Bestimmung des Schlagvolumens (SV) mittels Impedanzkardiographie wird ein vereinfachtes geometrisches Modell zur Darstellung des elektrischen Felds des Thorax verwendet, das nur stark näherungsweise die tatsächlichen Gegebenheiten widerspiegelt.

Während einer Herzperiode wird der Druck in der Aorta erhöht. Aufgrund der Elastizität der Aortawände wird daher auch der Durchmesser der Aorta vergrößert, wodurch die Impedanz des Thorax sinkt. Dieser Zusammenhang zwischen der Änderung der Impedanz des Thorax bei jedem Herzschlag und dem zentralen arteriellen Druck wurde bisher noch nicht zur Blutdruckmessung verwendet.

Bei der Blutdruckmessung ermittelt man mit Hilfe eines technischen Verfahrens den Druck in einem Blutgefäß. Man unterscheidet die Messung des arteriellen Drucks, des venösen Drucks, sowie die Messung in der Lungenschlagader (pulmonalarterieller Druck) und im Lungenkapillargebiet (pulmonalkapillärer Druck). Während die meisten Methoden spezielle Untersuchungsverfahren benötigen und teilweise Spezialverfahren außerhalb der Routine sind, spielt die Messung des arteriellen Druckes eine wichtige Rolle im medizinischen Alltag, da sie leicht durchführbar ist.

Man unterscheidet die direkte, invasive Druckmessung mittels eines Druckfühlers in einem Blutgefäß von der indirekten, nicht-invasiven Messung, die mit Hilfe einer Manschette an einer Extremität durchgeführt wird.

Bei der direkten, invasiven Messung (häufig mit "IBP", invasive blood pressure abgekürzt) wird ein Gefäß, zum Beispiel eine periphere Arterie, meist die Arteria radialis, punktiert und ein Drucksensor eingebracht. Über diesen lässt sich der Druckverlauf auf einem Monitor darstellen. Die Messung ist genau und bietet den Vorteil einer kontinuierlichen Überwachung, zusätzlich bestimmt das Gerät beim arteriellen Druck die Pulsfrequenz und den mittleren arteriellen Druck. Da die Methode invasiv ist, was mit dem Risiko von Blutungen, Infektionen und Nervenverletzungen einhergeht, wird sie vor allem von Anästhesisten zur Überwachung während einer Operation und auf Intensivstationen eingesetzt. Mit einer invasiven Druckmessung können auch der zentralvenöse Druck (in der oberen Hohlvene) und der pulmonalarterielle Druck (in der Lungenarterie) gemessen werden.

Bei der indirekten, nicht-invasiven arteriellen Druckmessung wird der arterielle Druck mit Hilfe eines Blutdruckmessgerätes an einer Extremität, meist am Arm, gemessen. Während die Messung auf diese Weise nicht so genau wie das direkte Verfahren ist, machen die leichte, schnelle, ungefährliche und kostengünstige Durchführung sie zum Mittel der Wahl in den meisten medizinischen Bereichen. Man unterscheidet die manuelle Messung von der automatischen Messung mittels eines digitalen Blutdruckmessgerätes. Wichtig dabei ist, dass die Manschette auf Herzhöhe ist, dies ist insbesondere bei Handgelenkgeräten zu beachten. Die manuelle Messung kann auskultatorisch, palpatorisch und oszillatorisch durchgeführt werden. Die Werte der einzelnen Methoden weichen dabei leicht voneinander ab.

Bei der auskultatorischen Messung wird eine Druckmanschette geeigneter Breite am Oberarm über den erwarteten arteriellen Druck aufgeblasen. Beim langsamen Ablassen kann man das Auftreten und danach wieder das Verschwinden eines Korotkow-Geräusches mit Hilfe eines Stethoskops über der Arterie des Armes hören. Der Druck, der bei Auftretensbeginn des gehörten Geräusches auf der Skala des Messgerätes abgelesen werden kann, entspricht dem oberen, systolischen arteriellen Druckwert, das heißt der systolische Druck ist in diesem Moment größer als der Druck der Manschette. Der Druck wird mit geeigneter Geschwindigkeit weiter abgelassen. Unterschreitet der Manschettendruck den minimalen arteriellen Druckwert, sistiert das Geräusch. Dieser Wert wird als diastolischer Druck bezeichnet und als sogenannter unterer Wert notiert. Die auskultatorische Messung ist das Standardverfahren der nicht-invasiven Messverfahren.

Auch bei der palpatorischen Messung wird eine Druckmanschette am Oberarm angelegt, beim Ablassen des Druckes wird der Puls an der Arteria radialis getastet. Der Druck, der beim erstmals getasteten Puls auf der Skala des Messgerätes abgelesen werden kann, entspricht dem oberen, systolischen arteriellen Druckwert. Der diastolische Wert kann auf diese Weise nicht ermittelt werden. Das Verfahren bietet sich für laute Umgebungen, insbesondere im Rettungsdienst, an.

Die oszillatorische Messung wird wie die beiden anderen Verfahren durchgeführt, der obere und untere Wert werden anhand des Amplitudenverlaufs eines pulsynchronen Zeigerausschlags am Messgerät abgeschätzt, welches die Übertragung von Schwingungen der Gefäßwand auf die Druckmanschette darstellt. Bei der manuellen Messung lassen sich so nur ungenaue Ergebnisse erzielen. Dieses Messprinzip wird jedoch zuverlässig von Messautomaten zur kontinuierlichen Überwachung, beispielsweise postoperativ im Aufwachraum eingesetzt. Diese messen als Alternative zur invasiven arteriellen Druckmessung den arteriellen Druck des Patienten im Intervall von wenigen Minuten. Das oszillatorische Messverfahren findet auch Anwendung in den mittlerweile weit verbreiteten Handgelenkmessgeräten.

Nachteilig an dieser Messmethode mittels Oberarmdruckmanschette ist allerdings, dass das kürzestmögliche Messintervall von wenigen Minuten insbesondere in der Intensivmedizin zu lange sein kann, und dass mit dieser Blutdruckmessung kein beat-to-beat Signal zur Verfügung steht. Insbesondere wenn hypotone Krisen, also Phasen mit zu niedrigem Blutdruck, nicht rechtzeitig erkannt werden, kann das post-operative Mortalitätsrisiko erheblich steigen. Eine nicht-invasive, kontinuierliche Blutdruckmessung, die Blutdruckabfälle verzögerungsfrei erkennen kann, ist also besonders erstrebenswert.

Eine Vorrichtung gemäß der Präambel des Anspruchs 1 ist in der Schrift US 2008/0009759 A1 offenbart.

Die vorliegende Erfindung stellt sich daher die Aufgabe, zur nicht-invasiven Bestimmung des arteriellen Blutdrucks die aus dem Stand der Technik bekannten Nachteile zu vermeiden, und dazu eine Vorrichtung zu schaffen, die gemäß dem Oberbegriff des Anspruches 1 mit den Merkmalen des kennzeichnenden Teiles des Anspruchs 1 ausgeführt ist.

Die Unteransprüche betreffen besonders vorteilhafte Ausgestaltungen der Erfindung.

Vorteilhaft umfasst eine erfindungsgemäße Vorrichtung zur nicht-invasiven Bestimmung des arteriellen Blutdrucks eines menschlichen oder tierischen Körpers, zumindest eine Bioimpedanz-Messeinrichtung mit mehreren Elektrodenpaaren zur Erfassung der durch einen eingeprägten Wechselstrom verursachten Admittanzmesssignale an zumindest einem ersten Abschnitt des Körpers, wobei die erfassten Admittanzmesssignale einem Summensignal aus Signalanteilen einer Puls-Admittanz, einer Atmungs-Admittanz sowie einer Basis-Admittanz entsprechen, sowie zumindest eine Einrichtung zur nicht-invasiven Blutdruckmessung, wobei eine Recheneinheit der Vorrichtung, die aus Admittanzmesssignalen, die von der Bioimpedanz-Messeinrichtung durch mehrere, im Abstand zueinander angeordnete Elektrodenpaare an zumindest einem ersten Abschnitt des Körpers erhalten werden, durch mehrfache Messungen mit jeweils unterschiedlich angeordneten Elektrodenpaaren und/oder bei jeweils unterschiedlichen Messfrequenzen zumindest die Signalanteile der Puls-Admittanz heraustrennt, sowie aus Drucksignalen eines vorzugsweise vom ersten Abschnitt beabstandeten zweiten Abschnitts des Körpers, die von der Blutdruckmesseinrichtung zur Bestimmung eines Skalierungsfaktors erhalten werden, den arteriellen Blutdruck bestimmt.

Standardgemäß wird zur Messung der Admittanz mit einem Impedanzkardiographie-Verfahren eine sogenannte 4-Draht-Messeinheit verwendet, bei der zumindest ein erstes Elektrodenpaar zum Einleiten eines kleinen, konstanten Messstroms in den Körper und zumindest ein weiteres, zweites Elektrodenpaar zum Abgreifen der Spannungsänderung vorgesehen sind. Beispielsweise werden streifenförmige Elektroden eingesetzt, wobei bei einer ersten Streifenelektrode der Strom eingeprägt wird und an der zumindest zweiten Streifenelektrode die Spannung gemessen wird. Beispielsweise wird beim Mensch ein erster Elektrodenstreifen im Bereich des Nacken geklebt, zumindest zwei weitere Elektrodenstreifen sind elektrisch parallel geschalten und werden am Thorax seitlich etwa in Höhe des Brustbeins angebracht.

Auch eine Anordnung der Elektroden nach Einthoven bietet sich zur Messung der Admittanz mit der erfindungsgemäßen Vorrichtung besonders an, da diese Elektrodenanordnung üblicherweise zur Bestimmung eines Elektrokardiogramm (EKG) verwendet wird und dem medizinischen Personal daher sehr geläufig ist. Außerdem wird mit der Anordnung nach Einthoven eine getrennte Erfassung der Signalanteile der Puls-Admittanz sowie der Atmungs-Admittanz erleichtert.

Biologische Gewebe haben jeweils einen charakteristischen, frequenzabhängigen Verlauf der Impedanz respektive der Admittanz. Es unterscheiden sich etwa die Impedanzverläufe von Lungengewebe und Blut. Diesen Umstand kann man nützen, um bei geeigneter Anordnung von mehreren Elektrodenpaaren bzw. bei mehrfacher Messung bei unterschiedlichen Messfrequenzen können die Anteile der Atmungs-Admittanz (dazu werden Elektroden möglichst nahe der Lunge positioniert) von der Puls-Admittanz (Elektroden werden dazu nahe dem Herz positioniert) zu trennen.

In der erfindungsgemäßen Vorrichtung wird von der Recheneinheit aus den Admittanzmesssignalen und den Drucksignalen der arterielle Blutdruck nach folgendem Schema bestimmt:

- Messen der mehreren Admittanzmesssignale an unterschiedlichen Körperstellen und/oder bei unterschiedlichen Messfrequenzen;
- Filtern der Admittanzmesssignale mit einem Hochpassfilter, sodass die niederfrequenten Anteile der Basis-Admittanz abgetrennt und gefilterte Admittanzsignale erhalten werden;

- Aufstellen von Summengleichungen, die Anteilsfaktoren der Puls-Admittanz sowie der Atmungs-Admittanz an den gefilterten Admittanzsignalen berücksichtigen;
- Verwenden eines Quellentrennungs-Algorithmus, um die Anteilsfaktoren sowie die Anteile der Quellensignale der Puls-Admittanz und der Atmungs-Admittanz aus den Summengleichungen zu bestimmen;
- Messen von Drucksignalen als Referenzdrucksignale;
- Bestimmen eines Skalenfaktors und eines Offset-Wertes, sodass die Puls-Admittanz mit den als Referenzdrucksignalen gemessenen Drucksignalen übereinstimmt;
- Nach Beendigen der Referenzmessung wird aus der Summe des Offset-Wertes mit dem Produkt aus Skalenfaktor und Puls-Admittanz der arterielle Blutdruck kontinuierlich bestimmt.

Insbesondere die Bestimmung von Drucksignalen beispielsweise einer Oberarmdruckmessung als Referenzsignale bietet beim Einsatz der erfindungsgemäßen Vorrichtung wesentliche Vorteile gegenüber einer konventionellen Oberarmdruckmessung. Eine konventionelle Oberarmdruckmessung dauert üblicherweise zumindest 30 Sekunden. Während dieser Zeitspanne werden der systolische Druckwert zu Beginn der Messung bei hohem Manschettendruck sowie der diastolische Druckwert am Ende der Messung bei niedrigem Manschettendruck bestimmt. Innerhalb dieser Zeitspanne kann sich jedoch der Blutdruck physiologisch signifikant verändern, was mit einer konventionellen Oberarmdruckmessung nicht erfassbar ist.

Mit der Puls-Admittanz liegt aber bei Einsatz der erfindungsgemäßen Vorrichtung vorteilhaft permanent ein relatives Maß für eine mögliche Änderung des Blutdrucks vor, das beispielsweise in eine oszillometrische Druckmessung einfließen kann.

Eine weitere vorteilhafte Ausführungsform der Erfindung umfasst eine Vorrichtung, wobei die Recheneinheit aus Admittanzmesssignalen, die von der Bioimpedanz-Messeinrichtung an zumindest einem ersten Abschnitt des Körpers erhalten werden, unter Verwendung eines Ausbreitungsalgorithmus, der die Ausbreitung von Druckwellen im Körper simuliert, aus den Admittanzmesssignalen zumindest die jeweiligen Signalanteile der Puls-Admittanz nach dem folgenden Schema heraustrennt:

- Filtern der Admittanzmesssignale mit einem Hochpassfilter, sodass die niederfrequenten Anteile abgetrennt werden und ein Signal einer gefilterten Admittanz erhalten wird;
- Filtern der Drucksignale mit dem Hochpassfilter, sodass ein gefiltertes Drucksignal erhalten wird;
- Anwenden eines adaptiven Filters, um eine Übertragungsfunktion vom gefilterten Drucksignal zur gefilterten Admittanz zu bestimmen;
- Anwenden eines Ausbreitungsalgorithmus, um eine extrapolierte Übertragungsfunktion zu bestimmen;
- Verwenden eines Optimierungsalgorithmus, um beispielsweise nach der Methode der kleinsten Fehlerquadrate einen Parametervektor des verwendeten Ausbreitungsalgorithmus sowie den Skalierungsfaktor zu bestimmen, sodass der Fehler zwischen der gefilterten Übertragungsfunktion und dem Produkt des Skalierungsfaktors mit der extrapolierten Übertragungsfunktion in einem höherfrequenten Messbereich minimal wird;
- Bestimmen der Puls-Admittanz unter Verwendung des Produkts des Skalierungsfaktors mit der extrapolierten Übertragungsfunktion und der Drucksignale;
- Bestimmen des arteriellen Blutdrucks mittels der extrapolierten Übertragungsfunktion und der Drucksignale.

Durch Filtern der niederfrequenten Anteile wird insbesondere der Anteil der Basis-Admittanz, also der Anteil des umgebenden Gewebes des Körpers, abgetrennt. Die Übertragungsfunktion, die unter Anwendung eines adaptiven Filters als funktionaler Zusammenhang zwischen einem gefilterten Drucksignal und der gefilterten Admittanz bestimmt wird, liefert nur in jenen Frequenzbereichen zuverlässige Schätzungen, in denen sowohl das jeweilige Eingangs-, als auch das Ausgangssignal jeweils ausreichend leistungsstark erfassbar ist. Im niederfrequenten Messbereich ist dies nicht der Fall. Daher muss eine weitere, auf den gesamten Frequenzbereich extrapolierte Übertragungsfunktion ermittelt werden, die anschließend auf die ungefilterten, gemessenen Drucksignale angewendet wird und derart einen Schätzwert der Puls-Admittanz liefert.

Durch Verwendung geeigneter Algorithmen erfolgt die Bestimmung des arteriellen Blutdrucks in Echtzeit als beat-to-beat-Bestimmung.

Zweckmäßig wird bei einer erfindungsgemäßen Vorrichtung als Ausbreitungsalgorithmus ein Transmission-line-Modell verwendet, wobei der Parametervektor einen Strömungswiderstandsparameter, einen Widerstandsparameter der Blutmasse gegen Beschleunigung, einen Elastizitätsparameter der Arterienwand sowie einen Porositätsparameter umfasst.

Das Transmission-line-Modell beschreibt im Grunde die Ausbreitung von elektromagnetischen Wellen in Leitungen. Durch biophysikalische Annahmen kann mit diesem Modell auch die Ausbreitung von Volumen- und Druckpulsen in elastischen Gefäßen beschrieben werden. Das Transmission-line-Modell besteht gedanklich aus einer Hintereinanderschaltung von infinitesimal kleinen, identischen Abschnitten, die jeweils durch Parameter des Parametervektors beschrieben werden. Wenn beim Transmission-line-Modell der Parametervektor bestimmt ist und somit der Ausbreitungsalgorithmus bekannt, kann mit diesem Modell vorteilhaft der arterielle Blutdruck entlang der gesamten Übertragungslinie bestimmt werden. Wenn also beispielsweise der zentrale Blutdruck und der periphere Blutdruck am Finger gemessen sind, kann auch ein brachialer Blutdruck am Oberarm bestimmt werden.

Weiters ist von Vorteil, dass mit dem Transmission-line Modell bei bekanntem Ausbreitungsalgorithmus durch einen Abgleich der berechneten Drucksignale mit den tatsächlich gemäßen Drucksignalen eine verbesserte Kalibrierung von zentralen, radialen sowie peripheren (brachialen) Blutdrucksignalen erzielt wird. Ein behandelnder Arzt kann somit beliebig jedes dieser Drucksignale von der erfindungsgemäßen Vorrichtung abrufen.

Vorteilhaft erfolgt bei einer Ausführung der erfindungsgemäßen Vorrichtung die nicht-invasive Blutdruckmesseinrichtung diskontinuierlich.

Die Blutdruckmesseinrichtung umfasst dazu beispielsweise eine Druckmanschette zum Anlegen an einer Körperextremität. Meist wird dazu der Oberarm verwendet.

Besonders zweckmäßig erfolgt in einer Ausführungsvariante der Vorrichtung die nicht-invasive Blutdruckmesseinrichtung kontinuierlich.

Die Blutdruckmesseinrichtung umfasst dazu beispielsweise eine Finger-Manschette zur kontinuierlichen Druckmessung. Vorteilhaft steht somit auch während einer Operation permanent ein aktuelles, kontinuierlich gemessenes Blutdrucksignal zur Verfügung, eine Oberarm-Druckmessung kann bei Messung des Blutdrucks mittels einer Finger-Manschette entfallen.

Weitere Merkmale der Erfindung ergeben sich aus der nachfolgenden Beschreibung von Ausführungsbeispielen und unter Bezugnahme auf die Zeichnungen.

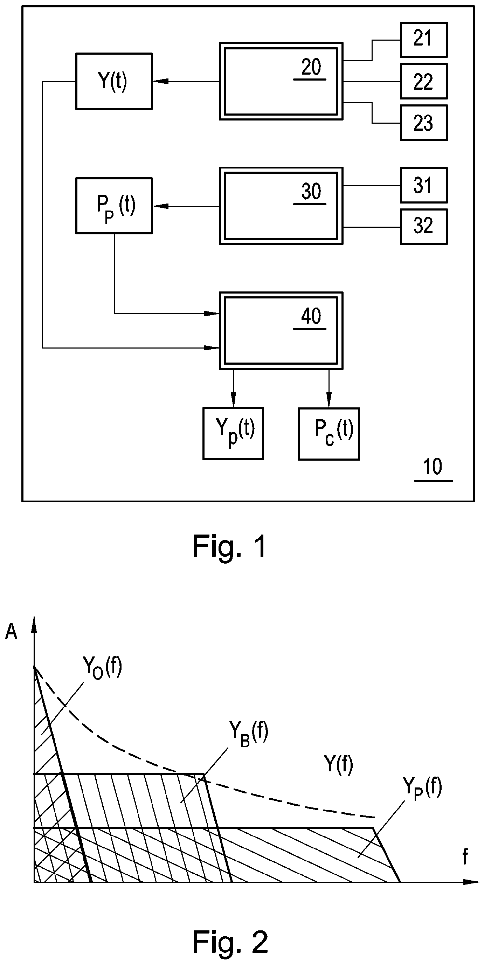

Fig. 1 zeigt schematisch vereinfacht eine erfindungsgemäße Vorrichtung 10 mit einer Bioimpedanz-Messeinrichtung 20 mit mehreren Elektrodenpaaren 21, 22, 23 zur Erfassung der durch einen eingeprägten Wechselstrom verursachten Admittanzmesssignale $Y(t)$ an zumindest einem ersten Abschnitt eines nicht dargestellten menschlichen Körpers, wobei die erfassten Admittanzmesssignale $Y(t)$, wenn sie am Thorax erhalten werden, einem Summensignal aus Signalanteilen einer Puls-Admittanz $Y_P(t)$, einer Atmungs-Admittanz $Y_B(t)$ sowie einer Basis-Admittanz $Y_0(t)$ entsprechen. Weiters umfasst die Vorrichtung 10 zumindest eine Einrichtung zur nicht-invasiven Blutdruckmessung 30, die mit einem Fingermanschetten-Druckmessgerät 31 zur Erfassung des peripheren Pulses $P_P(t)$ an einem Finger sowie mit einem Druckmessgerät 32 mit Oberarm-Manschette zur Erfassung der systolischen bzw. diastolischen Wertepaare des peripheren Pulses $P_P(t)$ am Oberarm des zu untersuchenden menschlichen Körpers ausgestattet ist.

Von einer Recheneinheit 40 der Vorrichtung 10 werden aus den Admittanzmesssignalen $Y(t)$, die von der Bioimpedanz-Messeinrichtung 20 durch die mehreren, im Abstand zueinander an zumindest einem ersten Abschnitt des Körpers angeordneten Elektrodenpaare 21, 22, 23 erhalten werden, zumindest die Signalanteile der Puls-Admittanz $Y_P(t)$ herausgetrennt. Weiters wird aus Drucksignalen $P_P(t)$ eines vorzugsweise vom ersten Abschnitt beabstandeten zweiten Abschnitts des Körpers, die von der Blutdruckmesseinrichtung 30 erhalten werden, ein Skalierungsfaktor k bestimmt. Der arterielle Blutdruck $P_C(t)$ wird daraufhin aus der Puls-Admittanz $Y_P(t)$ bestimmt.

Die erfindungsgemäße Vorrichtung 10 ist zur Bestimmung des arteriellen Blutdrucks $P_C(t)$ sowohl beim Mensch, als auch bei Tieren prinzipiell geeignet. Weiters kann die Vorrichtung 10 durch geeignete Elektrodenwahl auch zur Bestimmung des lokalen Blutdrucks beispielsweise an einem Bein eingesetzt werden.

Fig. 2 zeigt in einer schematischen Darstellung in Diagrammform die Überlagerung der Signalanteile der Puls-Admittanz $Y_P(f)$, der Atmungs-Admittanz $Y_B(f)$ sowie der Basis-Admittanz $Y_0(f)$ zum Summensignal der messbaren Admittanz $Y(f)$ - jeweils als Funktion der Frequenz f, die auf der Abszisse des Diagramms aufgetragen ist. Als Ordinatenwert ist hier die Amplitude A aufgetragen. Die Basis-Admittanz $Y_0(t)$ - im Wesentlichen durch das Körpergewebe bedingt - entspricht einem niederfrequenten Grundanteil bei hoher Amplitude. Diese wird von der Atmungs-Admittanz $Y_B(t)$ bis zu mittleren Frequenzen sowie der Puls-Admittanz $Y_P(t)$ bis zu hohen Frequenzen überlagert.

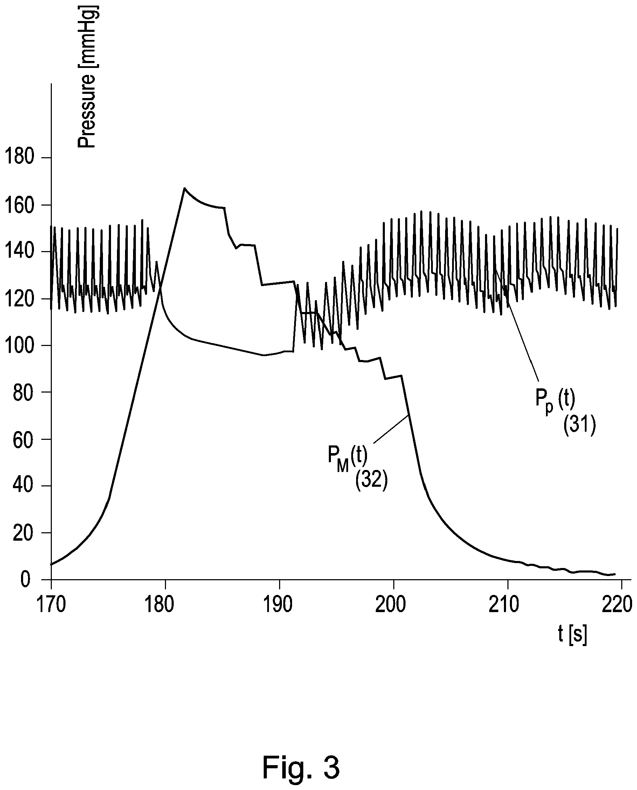

Fig. 3 zeigt in Diagrammform die beiden überlagerten peripheren Drucksignale $P_p(t)$ eines Druckmessgeräts 31 mit Finger-Manschette mit dem Drucksignal $P_M(t)$ eines Druckmessgeräts 32 mit Oberarm-Manschette. Die Drucksignale $P_P(t)$ bzw. $P_M(t)$ werden jeweils am selben Arm erfasst. Im Diagramm ist auf der Abszisse die Messdauer t (in Sekunden s) sowie auf der Ordinate der Blutdruck (in mm Hg) aufgetragen. Auffällig ist, dass das kontinuierliche Signal $P_p(t)$ des Druckmessgeräts 31 während der Messung des Druckmessgeräts 32 mit Oberarm-Manschette verfälscht wird. Die erfindungsgemäße Vorrichtung überwindet diesen Nachteil des Druckmessgeräts 32 mit Oberarm-Manschette, da der arterielle Blutdruck mit der erfindungsgemäßen Vorrichtung unterbrechungsfrei bestimmt wird.

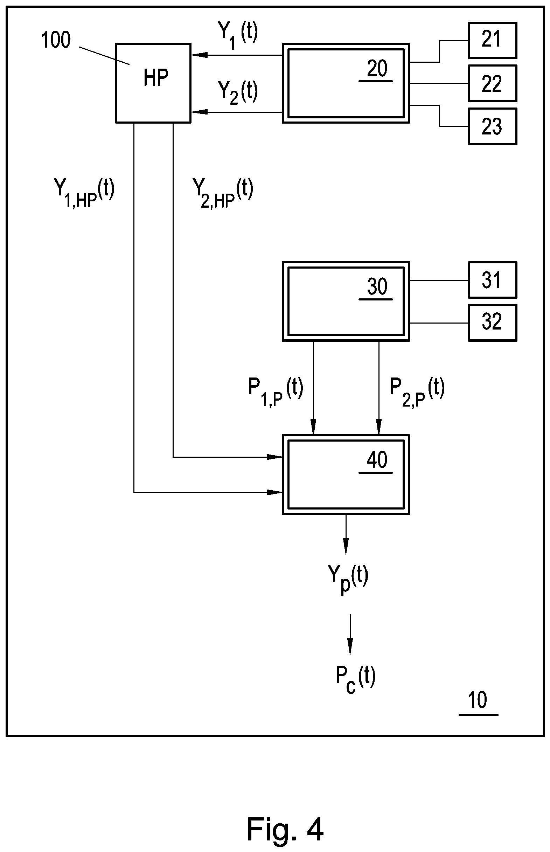

Fig. 4 zeigt schematisch vereinfacht eine erfindungsgemäße Vorrichtung 10 mit einer Bioimpedanz-Messeinrichtung 20 mit mehreren Elektrodenpaaren 21, 22, 23 zur Erfassung der durch einen eingeprägten Wechselstrom verursachten Admittanzmesssignale $Y_1(t)$ sowie

$Y_2(t)$ an zumindest einem ersten Abschnitt eines nicht dargestellten menschlichen Körpers. Die Admittanzsignale $Y_1(t)$ sowie $Y_2(t)$ wurden bei unterschiedlichen Messfrequenzen erhalten. wobei die erfassten Admittanzmesssignale $Y(t)$, wenn sie am Thorax erhalten werden, jeweils einem Summensignal aus Signalanteilen einer Puls-Admittanz $Y_P(t)$, einer Atmungs-Admittanz $Y_B(t)$ sowie einer Basis-Admittanz $Y_0(t)$ entsprechen.

Weiters umfasst die Vorrichtung 10 zumindest eine Einrichtung zur nicht-invasiven Blutdruckmessung 30, die mit einem Fingermanschetten-Druckmessgerät 31 zur Erfassung des peripheren Pulses $P_P(t)$ an einem Finger sowie mit einem Druckmessgerät 32 mit Oberarm-Manschette zur Erfassung des peripheren Pulses $P_P(t)$ am Oberarm des zu untersuchenden menschlichen Körpers ausgestattet ist. Es ist ausreichend, wenn die Vorrichtung 10 beispielsweise nur ein Druckmessgerät, entweder das Druckmessgerät 31, oder das Druckmessgerät 32, aufweist.

Die mehreren Admittanzmesssignale $Y_1(t)$ bzw. $Y_2(t)$ werden mit einem Hochpassfilter 100 gefiltert, sodass die niederfrequenten Anteile der Basis-Admittanz $Y_{1,0}(t)$ bzw. $Y_{2,0}(t)$ abgetrennt und von einer Recheneinheit 40 gefilterte Admittanzsignale $Y_{1,HP}(t)$ bzw. $Y_{2,HP}(t)$ erhalten werden. Von der Recheneinheit 40 werden Summengleichungen aufgestellt, die Anteilsfaktoren der Puls-Admittanz $Y_P(t)$ sowie der Atmungs-Admittanz $Y_B(t)$ an den gefilterten Admittanzsignalen berücksichtigen:

$$Y_{1,HP}(t) = k_{1,1} \cdot Y_B(t) + k_{1,2} \cdot Y_P(t) \quad \text{bzw.} \quad Y_{2,HP}(t) = k_{2,1} \cdot Y_B(t) + k_{2,2} \cdot Y_P(t);$$

Unter Verwendung eines Quellentrennungs-Algorithmus werden daraufhin von der Recheneinheit 40 die Anteilsfaktoren $k_{1,1}$, $k_{1,2}$, $k_{2,1}$, $k_{2,2}$ sowie die Quellensignalanteile der Puls-Admittanz $Y_P(t)$ und der Atmungs-Admittanz $Y_B(t)$ aus den Summengleichungen bestimmt.

Weiters werden Drucksignale $P_{1,P}(t)$ sowie $P_{2,P}(t)$) von der Einrichtung zur Blutdruckmessung 30 erfasst und als Referenzdrucksignale an die Recheneinheit 40 weiter geleitet. Bei Verwendung eines Druckmessgeräts 32 mit Oberarm-Manschette entsprechen die Drucksignale $P_{1,P}(t)$ und $P_{2,P}(t)$ beispielsweise einem zugeordneten Wertepaar aus systolischem sowie diastolischem Blutdruck. Anschließend werden von der Recheneinheit 40 ein Skalenfaktor k und ein Offset-Wert d bestimmt, sodass die Puls-Admittanz $Y_P(t)$ mit den als Referenzdrucksignalen gemessenen Drucksignalen $P_{1,P}(t)$ sowie $P_{2,P}(t)$ übereinstimmt. Der Zusammenhang der einzelnen Rechengrößen ist beispielsweise wie folgt:

$$P_{1,P}(t) = k \cdot Y_{P,Sys} + d \quad \text{bzw.} \quad P_{2,P}(t) = k \cdot Y_{P,Dia} + d$$

Nach Beendigen der Referenzmessung wird der arterielle Blutdruck $P_C(t)$ von der Recheneinheit 40 der Vorrichtung 10 kontinuierlich unter Verwendung des Skalenfaktors k und des Offset-Wertes d gemäß der Gleichung $P_C(t) = k \cdot Y_P(t) + d$ bestimmt.

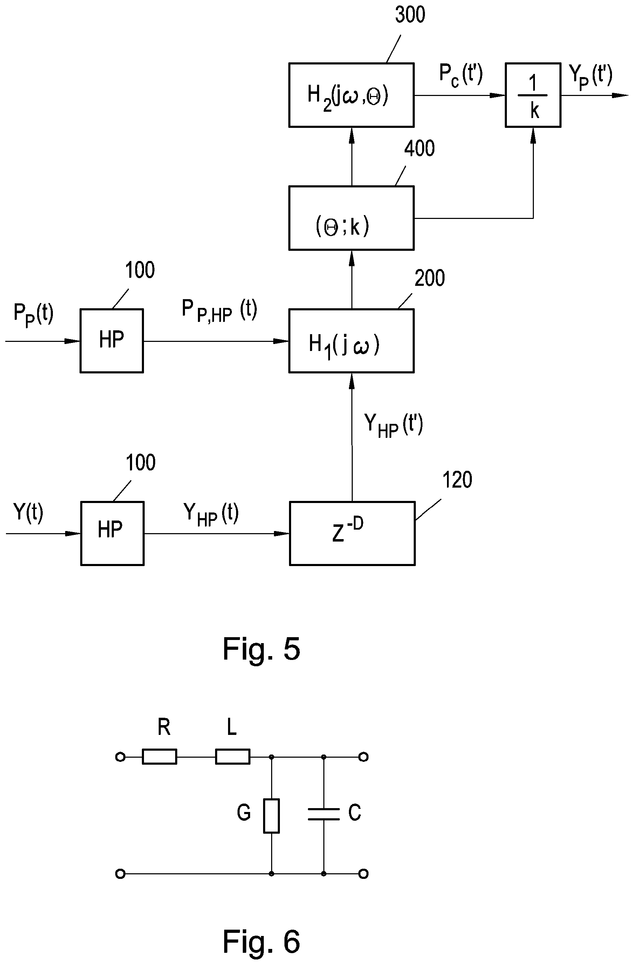

Fig. 5 stellt in einer schematischen Darstellung ein vereinfachtes Ablaufschema zur Bestimmung des arteriellen Blutdrucks $P_C(t)$ unter Verwendung eines Ausbreitungsalgorithmus.

Von der Bioimpedanz-Messeinrichtung der erfindungsgemäßen Vorrichtung werden Admittanzmesssignale $Y(t)$ erfasst, die anschließend mit einem Hochpassfilter 100 gefiltert werden, sodass die niederfrequenten Signalanteile abgetrennt und Signale einer gefilterten Admittanz $Y_{HP}(t)$ erhalten werden. Da das gemessene periphere Drucksignal $P_P(t)$ jeweils dem zu bestimmenden, zentralen arteriellen Drucksignal $P_C(t)$ nacheilt, ist es erforderlich, die Übertragung des gemessenen Admittanzsignals $Y(t)$ bzw. des korrespondierenden Signals der gefilterten Admittanz $Y_{HP}(T)$ zeitlich etwas zu verzögern und derart eine zeitliche Korrelation des Ausbreitungsalgorithmus vorzusehen. Dazu wird eine Verzögerungsleitung 120 vorgesehen, die das gefilterte Admittanzsignal $Y_{HP}(t')$ gegenüber dem Admittanzsignal $Y_{HP}(t)$ verzögert an das adaptive Filter 200 weiterleitet. Der Zeitpunkt t' ist dabei geringfügig gegenüber dem Zeitpunkt t verzögert.

Die von der Einrichtung zur Blutdruckmessung der Vorrichtung erfassten Drucksignale Pp(t) werden ebenso mit einem Hochpassfilter 100 gefiltert, worauf ein gefiltertes Drucksignal $P_{P,HP}(t)$ erhalten wird. Ein adaptives Filter 200 wird verwendet, um eine gefilterte Übertragungsfunktion $H_1(j\omega)$ vom gefilterten Drucksignal $P_{P,HP}(t)$ zur gefilterten Admittanz $Y_{HP}(j\omega)$ zu bestimmen.

Weiters wird ein Ausbreitungsalgorithmus 300 angewandt, um eine extrapolierte Übertragungsfunktion $H_2(j\omega,\theta)$ zu bestimmen. Dazu wird ein Optimierungsalgorithmus 400 verwendet, um beispielsweise nach der Methode der kleinsten Fehlerquadrate einen Parametervektor 0 des verwendeten Ausbreitungsalgorithmus 300 sowie den Skalierungsfaktor k zu bestimmen, sodass der Fehler zwischen der gefilterten Übertragungsfunktion $H_1(j\omega)$ und dem Produkt des Skalierungsfaktors k mit der extrapolierten Übertragungsfunktion $H_2(j\omega,\theta)$ in einem höherfrequenten Messbereich minimal wird.

Der arterielle Blutdruck $P_C(t)$ wird mittels der extrapolierten Übertragungsfunktion $H_2(j\omega,\theta)$ und des Drucksignales $P_P(t)$ bestimmt. Die Puls-Admittanz $Y_P(t)$ zur Rekonstruktion des Anteils der Atmungs-Admittanz $Y_B(t)$ wird unter Verwendung des Produkts des Skalierungsfaktors k mit der extrapolierten Übertragungsfunktion $H_2(j\omega,\theta)$ und der Drucksignale $P_P(t)$ erhalten.

Fig. 6 zeigt schematisch das Ersatzschaltbild des Transmission-line-Modells als Ausbreitungsalgorithmus. Der

Parametervektor ($\theta$) des Modells umfasst einen Strömungswiderstandsparameter (R), einen Widerstandsparameter (L) der Blutmasse gegen Beschleunigung, einen Elastizitätsparameter (C) der Arterienwand sowie einen Porositätsparameter (G).

**Patentansprüche**

1. Vorrichtung (10) zur nicht-invasiven Bestimmung des arteriellen Blutdrucks eines menschlichen oder tierischen Körpers, umfassend zumindest eine Bioimpedanz-Messeinrichtung (20) mit mehreren Elektrodenpaaren (21, 22, 23) zur Erfassung der durch einen eingeprägten Wechselstrom verursachten Admittanzmesssignale (Y(t)) an zumindest einem ersten Abschnitt des Körpers, wobei die erfassten Admittanzmesssignale (Y(t)) einem Summensignal aus Signalanteilen einer Puls-Admittanz ($Y_P$(t)), einer Atmungs-Admittanz ($Y_B$(t)) sowie einer Basis-Admittanz ($Y_0$(t)) entsprechen, sowie zumindest eine Einrichtung zur nicht-invasiven Blutdruckmessung (30), wobei eine Recheneinheit (40) der Vorrichtung (10) ausgelegt ist, die aus Admittanzmesssignalen (Y(t)), die von der Bioimpedanz-Messeinrichtung (20) durch mehrere, im Abstand zueinander angeordnete Elektrodenpaare (21, 22, 23) an zumindest einem ersten Abschnitt des Körpers erhalten werden, wobei die Messungen mehrfach mit jeweils unterschiedlich am Körper angeordneten Elektrodenpaaren und/oder bei jeweils unterschiedlichen Messfrequenzen erfolgen, zumindest die Signalanteile der Puls-Admittanz ($Y_P$(t)) herauszutrennen, sowie aus Drucksignalen ($P_P$(t)) eines vom ersten Abschnitt beabstandeten zweiten Abschnitts des Körpers, die von der Blutdruckmesseinrichtung erhalten werden, einen Skalierungsfaktor (k) und den arteriellen Blutdruck ($P_C$(t)) zu bestimmen, **dadurch gekennzeichnet, dass** die Recheneinheit (40) weiterhin ausgelegt ist, den arteriellen Blutdruck ($P_C$(t)) nach folgendem Schema zu bestimmen:

    - Messen der mehreren Admittanzmesssignale ($Y_1$(t), $Y_2$(t)) an unterschiedlichen Körperstellen und/oder bei unterschiedlichen Messfrequenzen;
    - Filtern der Admittanzmesssignale (($Y_1$(t), $Y_2$(t)) mit einem Hochpassfilter (100), sodass die niederfrequenten Anteile der Basis-Admittanz ($Y_{1,0}$(t), $Y_{2,0}$(t)) abgetrennt und gefilterte Admittanzsignale ($Y_{1,HP}$(t), $Y_{2,HP}$(t)) erhalten werden;
    - Aufstellen von Summengleichungen, die Anteilsfaktoren der Puls-Admittanz ($Y_P$(t)) sowie der Atmungs-Admittanz ($Y_B$(t)) an den gefilterten Admittanzsignalen berücksichtigen:

$$Y_{1,HP}(t) = k_{1,1} \cdot Y_B(t) + k_{1,2} \cdot Y_P(t)$$

$$Y_{2,HP}(t) = k_{2,1} \cdot Y_B(t) + k_{2,2} \cdot Y_P(t);$$

    - Verwenden eines Quellentrennungs-Algorithmus, um die Anteilsfaktoren ($k_{1,1}$, $k_{1,2}$, $k_{2,1}$, $k_{2,2}$) sowie die Quellensignalanteile der Puls-Admittanz ($Y_P$(t)) und der Atmungs-Admittanz ($Y_B$(t)) aus den Summengleichungen zu bestimmen;
    - Messen von Drucksignalen ($P_{1 \cdot P}$(t), $P_{2,P}$(t)) als Referenzdrucksignale;
    - Bestimmen des Skalierungsfaktors (k) und eines Offset-Wertes (d), sodass die Puls-Admittanz ($Y_P$(t)) mit den als Referenzdrucksignalen gemessenen Drucksignalen ($P_{1,P}$(t), $P_{2,P}$(t)) übereinstimmt:

$$P_{1,P}(t) = k \cdot Y_{P,Sys} + d$$

$$P_{2,P}(t) = k \cdot Y_{P,Dia} + d;$$

    - Nach Beendigen der Referenzmessung wird der arterielle Blutdruck ($P_C$(t)) kontinuierlich unter Verwendung des Skalierungsfaktors (k) und des Offset-Wertes (d) gemäß

$$P_C(t) = k \cdot Y_P(t) + d$$

bestimmt.

**2.** Vorrichtung nach Anspruch 1, **dadurch gekennzeichnet, dass** die Recheneinheit weiterhin ausgelegt ist, aus Admittanzmesssignalen (Y(t)), die von der Bioimpedanz-Messeinrichtung an zumindest einem ersten Abschnitt des Körpers erhalten werden, unter Verwendung eines Ausbreitungsalgorithmus, der die Ausbreitung von Druckwellen im Körper simuliert, aus den Admittanzmesssignalen (Y(t)) zumindest die jeweiligen Signalanteile der Puls-Admittanz ($Y_P$(t)) nach dem folgenden Schema herauszutrennen:

- Filtern der Admittanzmesssignale (Y(t)) mit einem Hochpassfilter (100), sodass die niederfrequenten Anteile abgetrennt werden und ein Signal einer gefilterten Admittanz ($Y_{HP}$(t)) erhalten wird;
- Filtern der Drucksignale ($P_P$(t)) mit einem Hochpassfilter (100), sodass ein gefiltertes Drucksignal ($P_{P,HP}$(t)) erhalten wird;
- Anwenden eines adaptiven Filters (200), um eine gefilterte Übertragungsfunktion ($H_1$(jω)) vom gefilterten Drucksignal ($P_{P,HP}$(t)) und von der gefilterten Admittanz ($Y_{HP}$(jω)) zu bestimmen;
- Anwenden eines Ausbreitungsalgorithmus (300), um eine extrapolierte Übertragungsfunktion ($H_2$(jω,θ)) zu bestimmen;
- Verwenden eines Optimierungsalgorithmus (400), um beispielsweise nach der Methode der kleinsten Fehlerquadrate einen Parametervektor (θ) des verwendeten Ausbreitungsalgorithmus (300) sowie den Skalierungsfaktor (k) zu bestimmen, sodass der Fehler zwischen der gefilterten Übertragungsfunktion ($H_1$(jω)) und dem Produkt des Skalierungsfaktors (k) mit der extrapolierten Übertragungsfunktion ($H_2$(jω,θ)) in einem höherfrequenten Messbereich minimal wird;
- Bestimmen der Puls-Admittanz ($Y_P$(t)) unter Verwendung des Produkts des invertierten Skalierungsfaktors (k) mit der extrapolierten Übertragungsfunktion ($H_2$(jω,θ)) und der Drucksignale ($P_P$(t));
- Bestimmen des arteriellen Blutdrucks ($P_C$(t)) mittels der extrapolierten Übertragungsfunktion ($H_2$(jω,θ)) und der Drucksignale ($P_P$(t)).

**3.** Vorrichtung nach Anspruch 2, **dadurch gekennzeichnet, dass** als Ausbreitungsalgorithmus ein Transmission-line-Modell verwendet wird, wobei der Parametervektor (θ) einen Strömungswiderstandsparameter (R), einen Widerstandsparameter (L) der Blutmasse gegen Beschleunigung, einen Elastizitätsparameter (C) der Arterienwand sowie einen Porositätsparameter (G) umfasst.

**4.** Vorrichtung nach einem der Ansprüche 1 bis 3, **dadurch gekennzeichnet, dass** die nicht-invasive Blutdruckmesseinrichtung diskontinuierlich erfolgt.

**5.** Vorrichtung nach einem der Ansprüche 1 bis 3, **dadurch gekennzeichnet, dass** die nicht-invasive Blutdruckmesseinrichtung kontinuierlich erfolgt.

**Claims**

**1.** A device (10) for non-invasive determination of the arterial blood pressure of a human or animal body, comprising at least one bioimpedance measuring device (20) having a plurality of electrode pairs (21, 22, 23) for detecting the admittance measuring signals (Y(t)) induced by an impressed alternating current on at least a first portion of the body, with the detected admittance measuring signals (Y(t)) corresponding to a sum signal made of signal components of a pulse admittance ($Y_p$(t)), a respiratory admittance ($Y_B$(t)) as well as a base admittance ($Y_0$(t)), and at least one apparatus for non-invasive blood pressure measurement (30), wherein a computing unit (40) of the device (10) is designed for separating at least the signal components of the pulse admittance ($Y_p$(t)) from admittance measuring signals (Y(t)) obtained by the bioimpedance measuring device (20) by a plurality of electrode pairs (21, 22, 23) spaced apart relative to each other on at least a first portion of the body, wherein the measurements are performed repeatedly with electrode pairs each arranged differently on the body and/or at measuring frequencies different in each case, and for determining a scaling factor (k) and the arterial blood pressure ($P_C$(t)) from pressure signals ($P_p$(t)) of a second portion of the body spaced apart from the first portion, which pressure signals are obtained from the blood pressure measuring device, **characterized in that** the computing unit (40) is furthermore designed for determining the arterial blood pressure ($P_C$(t)) according to the following scheme:

- measuring the plurality of admittance measuring signals ($Y_1$(t), $Y_2$(t)) on various body sites and/or at various measuring frequencies;
- filtering the admittance measuring signals (($Y_1$(t), $Y_2$(t)) by means of a high-pass filter (100) so that the low-frequency components of the base admittance ($Y_{1,0}$(t), $Y_{2,0}$(t)) are separated and filtered admittance signals

($Y_{1,HP}$(t), $Y_{2,HP}$(t)) are obtained;
- establishing sum equations which factor in proportional factors of the pulse admittance ($Y_P$(t)) as well as the respiratory admittance ($Y_B$(t)) at the filtered admittance signals:

$$Y_{1,HP}(t) = k_{1,1} \cdot Y_B(t) + k_{1,2} \cdot Y_P(t)$$

$$Y_{2,HP}(t) = k_{2,1} \cdot Y_B(t) + k_{2,2} \cdot Y_P(t);$$

- using a source separation algorithm for determining the proportional factors ($k_{1,1}$, $k_{1,2}$, $k_{2,1}$, $k_{2,2}$) as well as the source signal components of the pulse admittance ($Y_p$(t)) and the respiratory admittance ($Y_B$(t)) from the sum equations;
- measuring pressure signals ($P_{1,P}$(t), $P_{2,P}$(t)) as reference pressure signals;
- determining the scaling factor (k) and an offset value (d) so that the pulse admittance ($Y_p$(t)) corresponds to the pressure signals ($P_{1,P}$(t), $P_{2,P}$(t)) measured as reference pressure signals;

$$P_{1,P}(t) = k \cdot Y_{P,Sys} + d$$

$$P_{2,P}(t) = k \cdot Y_{P,Dia} + d;$$

- upon completion of the reference measurement, the arterial blood pressure ($P_C$(t)) is determined continuously using the scaling factor (k) and the offset value (d) according to

$$P_C(t) = k \cdot Y_P(t) + d.$$

2. A device according to claim 1, **characterized in that** the computing unit is furthermore designed for separating, from admittance measuring signals (Y(t)) obtained by the bioimpedance measuring device on at least a first portion of the body, at least the respective signal components of the pulse admittance ($Y_p$(t)) from the admittance measuring signals (Y(t)), using a propagation algorithm simulating the propagation of pressure waves within the body, according to the following scheme:

- filtering the admittance measuring signals (Y(t)) with a high-pass filter (100) so that the low-frequency components are separated and a signal of a filtered admittance ($Y_{HP}$(t)) is obtained;
- filtering the pressure signals ($P_P$(t)) with a high-pass filter (100) so that a filtered pressure signal ($P_{P,HP}$(t)) is obtained;
- applying an adaptive filter (200) so as to determine a filtered transfer function ($H_1$(jω)) from the filtered pressure signal ($P_{P,HP}$(t)) and from the filtered admittance ($Y_{HP}$(jω));
- applying a propagation algorithm (300) so as to determine an extrapolated transfer function ($H_2$(jω,θ));
- using an optimization algorithm (400) for determining a parameter vector (θ) of the applied propagation algorithm (300) as well as the scaling factor (k), for example, according to the method of least error squares so that the error between the filtered transfer function ($H_1$(jω)) and the product of the scaling factor (k) with the extrapolated transfer function ($H_2$(jω,θ)) becomes minimal in a higher-frequency measuring range;
- determining the pulse admittance ($Y_P$(t)) using the product of the inverted scaling factor (k) with the extrapolated transfer function ($H_2$(jω,θ)) and the pressure signals ($P_P$(t));
- determining the arterial blood pressure ($P_C$(t)) by means of the extrapolated transfer function ($H_2$(jω,θ)) and the pressure signals ($P_P$(t)).

3. A device according to claim 2, **characterized in that** a transmission-line model is used as the propagation algorithm, wherein the parameter vector (θ) comprises a flow resistance parameter (R), a resistance parameter (L) of the blood mass against acceleration, an elasticity parameter (C) of the arterial wall as well as a porosity parameter (G).

4. A device according to any of claims 1 to 3, **characterized in that** the non-invasive blood pressure measuring device is discontinuous.

**5.** A device according to any of claims 1 to 3, **characterized in that** the non-invasive blood pressure measuring device is continuous.

**Revendications**

**1.** Dispositif (10) destiné à déterminer de manière non invasive la tension artérielle d'un corps humain ou animal, comprenant au moins un moyen de mesure de bio-impédance (20) avec plusieurs paires d'électrodes (21, 22, 23) pour déterminer des signaux de mesure d'admittance (Y(t)) produits par un courant alternatif appliqué sur au moins une première section du corps, dans lequel les signaux de mesure d'admittance déterminés (Y(t)) correspondent à un signal de sommation de parties de signaux d'une admittance de pouls ($Y_P(t)$), d'une admittance de respiration ($Y_B(t)$) ainsi que d'une admittance de base ($Y_0(t)$) et au moins un moyen (30) de mesure de la tension artérielle de manière non invasive, dans lequel une unité de calcul (40) du dispositif (10) est conçue pour séparer au moins les parties de signaux de l'admittance de pouls ($Y_P(t)$) des signaux de mesure d'admittance (Y(t)), qui ont été obtenus par le moyen de mesure de bio-impédance (20) par le biais de plusieurs paires d'électrodes (21, 22, 23) disposées à distance l'une de l'autre sur au moins une première section du corps, dans lequel les mesures sont réalisées plusieurs fois avec à chaque fois des paires d'électrodes disposées de manière différente sur le corps et/ou avec des fréquences de mesure à chaque fois différentes, ainsi que pour définir un facteur d'échelle (k) et la tension artérielle ($P_C(t)$) à partir de signaux de pression ($P_P(t)$) d'une seconde section du corps située à distance de la première section, qui ont été obtenus par le moyen de mesure de tension artérielle, **caractérisé en ce que** l'unité de calcul (40) est en outre conçu pour définir la tension artérielle ($P_C(t)$) selon le schéma suivant :

- mesurer plusieurs signaux de mesure d'admittance ($Y_1(t)$, $Y_2(t)$) en différents endroits du corps et/ou avec des fréquences de mesure différentes ;
- filtrer les signaux de mesure d'admittance (($Y_1(t)$, $Y_2(t)$) avec un filtre passe-haut (100), de manière à séparer les parties à basse fréquence de l'admittance de base ($Y_{1,0}(t)$, $Y_{2,0}(t)$) et à obtenir des signaux d'admittance filtrés ($Y_{1,HP}(t)$, $Y_{2,HP}(t)$) ;
- former des équations de sommation, les facteurs de parties de l'admittance de pouls ($Y_P(t)$) et de l'admittance de respiration ($Y_B(t)$) prenant en compte, au niveau des signaux d'admittance filtrés :

$$Y_{1,HP}(t) = k_{1,1} \cdot Y_B(t)) + k_{1,2} \cdot Y_P(t))$$

$$Y_{2,HP}(t) = k_{2,1} \cdot Y_B(t)) + k_{2,2} \cdot Y_P(t)) ;$$

- utiliser un algorithme de séparation aveugle de sources, afin de définir des facteurs des parties ($k_{1,1}$, $k_{1,2}$, $k_{2,1}$, $k_{2,2}$) ainsi que les parties de signaux sources de l'admittance de pouls ($Y_P(t)$) et de l'admittance de respiration ($Y_B(t)$) à partir des équations de sommation ;
- mesurer les signaux de pression ($P_{1,P}(t)$, $P_{2,P}(t)$) en tant que signaux de pression de référence ;
- définir un facteur d'échelle (k) et une valeur d'écart (d), de sorte que l'admittance de pouls ($Y_P(t)$) coïncide avec les signaux de pression (($P_{1,P}(t)$, $P_{2,P}(t)$) mesurées en tant que signaux de pression de référence :

$$P_{1,P}(t) = k \cdot Y_{P,Sys} + d$$

$$P_{2,P}(t) = k \cdot Y_{P,Dia} + d ;$$

- après la fin de la mesure de référence, la tension artérielle ($P_C(t)$) est définie de manière continue comme suit, en utilisant le facteur d'échelle (k) et la valeur d'écart (d) :

$$P_C(t) = k \cdot Y_P(t) + d.$$

**2.** Dispositif selon la revendication 1, **caractérisé en ce que** l'unité de calcul est en outre conçue pour séparer au moins les parties de signaux respectives de l'admittance de pouls ($Y_P(t)$) des signaux de mesure d'admittance ($Y(t)$), qui ont été obtenus par le moyen de mesure de bio-impédance au niveau d'au moins une première section du corps, en utilisant un algorithme de propagation, qui simule la propagation d'ondes de pression dans le corps, des signaux de mesure d'admittance ($Y(t)$) selon le schéma suivant :

- filtrer les signaux de mesure d'admittance ($Y(t)$) avec un filtre passe-haut (100), de manière à séparer les parties à basse fréquence et à obtenir un signal d'une admittance filtrée ($Y_{HP}(t)$) ;
- filtrer les signaux de pression ($P_P(t)$) avec un filtre passe-haut (100), de manière à obtenir un signal de pression filtré ($P_{P,HP}(t)$) ;
- appliquer un filtre adaptatif (200), pour définir une fonction de transfert filtrée ($H_1(j\omega)$) du signal de pression filtré ($P_{P,HP}(t)$) et de l'admittance filtrée ($Y_{HP}(j\omega)$) ;
- appliquer un algorithme de propagation (300), pour définir une fonction de transfert extrapolée ($H_2(j\omega,\theta)$) ;
- utiliser un algorithme d'optimisation (400), pour définir, par exemple d'après la méthode des moindres carrés, un vecteur de paramètre ($\theta$) de l'algorithme de propagation utilisé (300) ainsi que le facteur d'échelle (k), de sorte que l'erreur entre la fonction de transfert filtrée ($H_1(j\omega)$) et le produit du facteur d'échelle (k) avec la fonction de transfert extrapolée ($H_2(j\omega,\theta)$) soit minime dans une plage de mesures à haute fréquence ;
- définir l'admittance de pouls ($Y_P(t)$) en utilisant le produit du facteur d'échelle (k) avec la fonction de transfert extrapolée ($H_2(j\omega,\theta)$) et des signaux de pression ($P_P(t)$) ;
- définir la tension artérielle ($P_C(t)$) à l'aide de la fonction de transfert extrapolée ($H_2(j\omega,\theta)$) et des signaux de pression ($P_P(t)$).

3. Dispositif selon la revendication 2, **caractérisé en ce qu'**on utilise un modèle de ligne de transmission pour servir d'algorithme de propagation, dans lequel le vecteur de paramètre ($\theta$) comprend un paramètre de résistance d'écoulement (R), un paramètre de résistance (L) de la masse sanguine contre l'accélération, un paramètre d'élasticité (C) de la paroi artérielle ainsi qu'un paramètre de porosité (G).

4. Dispositif selon l'une des revendications 1 à 3, **caractérisé en ce que** le moyen de mesure de tension artérielle non invasive est réalisé de manière discontinue.

5. Dispositif selon l'une des revendications 1 à 3, **caractérisé en ce que** le moyen de mesure de tension artérielle non invasive est réalisé de manière continue.

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080009759 A1